(19) Europäisches Patentamt
European Patent Office
Office européen des brevets

(11) EP 4 129 412 A1

(12) # EUROPÄISCHE PATENTANMELDUNG

(43) Veröffentlichungstag:
**08.02.2023 Patentblatt 2023/06**

(21) Anmeldenummer: **22182769.4**

(22) Anmeldetag: **04.07.2022**

(51) Internationale Patentklassifikation (IPC):
***A61Q 5/12*** *(2006.01)* ***A61K 8/06*** *(2006.01)*
***A61K 8/37*** *(2006.01)* ***A61K 8/41*** *(2006.01)*
***A61K 8/42*** *(2006.01)*

(52) Gemeinsame Patentklassifikation (CPC):
**A61Q 5/12; A61K 8/062; A61K 8/37; A61K 8/416; A61K 8/42;** A61K 2800/596

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **05.08.2021 DE 102021208512**

(71) Anmelder: **Beiersdorf AG**
**20245 Hamburg (DE)**

(72) Erfinder:
- **Reiter, Katharina**
  **21357 Barum (DE)**
- **Nemnich, Julia**
  **22589 Hamburg (DE)**

(74) Vertreter: **Beiersdorf AG**
**Patentabteilung**
**Unnastraße 48**
**20245 Hamburg (DE)**

(54) **PFLEGENDES LEAVE-IN-SPRAY FÜR DIE HAARE**

(57) Leave-in-Spray vorgesehen für die kosmetische Anwendung auf menschlichen Kopfhaaren, wobei die die Haare nicht beschwert, gleichwohl aber gut pflegt werden.

EP 4 129 412 A1

## Beschreibung

Die vorliegende Erfindung beschreibt ein Leave-in-Spray für die Haare, das die Haare nicht beschwert, gleichwohl aber gut pflegt.

Leave-in-Sprays werden in der Regel nach der Reinigung der Haare angewendet. Sie werden auf das nasse Haar aufgetragen, insbesondere aufgesprüht und können dann einmassiert werden. Eine Anwendung auf dem trockenen Haar ist auch vorteilhaft, verleiht sie dem Haar doch eine "Extra-Portion" Pflege zwischen den Haarwäschen.

Leave-in-Sprays für die Haare sind an sich bekannt, wie der StdT zeigt.

Ein Nourishing Leave-in Spray wird in der Mintel Datenbank unter der Eintragsnummer 3599435 beschrieben.

Weiterhin wird unter der Eintragsnummer 7424479 ein Repairing Leave-in Spray Treatment in der Mintel Datenbank offenbart.

Im Dokument EP 2783677 A2 werden Esterquats beschrieben, die auf Isopropanolamin basieren. In einigen Formulierungsbeispielen wurden diese Esterquats in Leave-in Conditioner Sprays eingearbeitet.

Durch eine Haarwäsche wird das Haar gereinigt, d.h. es werden Schmutzpartikel und/oder -filme, die von außen auf das Haar gelangt sind, entfernt. Gleichzeitig wird aber auch die äußere Schicht des Haares, die Cuticula, angegriffen. Die Cuticula weist auf der Oberfläche kovalent gebundene Fettsäuren auf, die zur Hydrophobizität der Cuticula beitragen. Durch den Waschvorgang können zumindest zu einem gewissen Teil diese Fettsäuren ausgewaschen werden. Dieser Prozess kann bewirken, dass das Haar trocken wird.

Um diesem Phänomen entgegenzuwirken, verwenden viele Verbraucher/innen einen Conditioner nach der Haarwäsche. Conditioner werden in der Regel wieder ausgewaschen. Auf diese Weise werden die pflegenden Komponenten wenigstens teilweise wieder ausgespült. Um diesen Nachteil zu überwinden können Leave-in-Sprays verwendet werden, die oft ähnliche pflegende Komponenten wie Conditioner enthalten. Die pflegenden Komponenten der Leave-in-Sprays haben somit eine verbesserte Möglichkeit ihren pflegenden Effekt zu entfalten, da sie dafür vorgesehen sind auf dem Haar zu verbleiben.

Der Zweck der Verwendung dieser Leave-in Sprays ist es, dem Haar idealerweise die Komponenten zurückzugeben, die möglicherweise durch die Haarwäsche mit ausgewaschen wurden. Deshalb sind im allgemeinen lipophile Komponenten in Leave-in-Sprays enthalten, die mit den lipophilen Verbindungen der Cuticula wechselwirken können.

Neben der Hydrophobizität der Cuticula, ist auch die Proteinstruktur der Cuticula zu beachten. Die Zusammensetzung der Proteinstruktur resultiert in einem isoelektrischen Punkt von etwa 3,67 an der Haaroberfläche. Der pH-Wert, den die üblicherweise eingesetzten Haar-kosmetische Zubereitungen aufweisen, bewirkt, dass negativ geladene Strukturen auf der Haaroberfläche vorliegen.

Pflegende Komponenten in Leave-in-Sprays umfassen also lipophile Verbindungen, die mit den lipophilen Verbindungen an der Cuticula-Oberfläche in Wechselwirkung treten und/oder positive geladene Verbindungen (kationische Verbindungen), die mit den negativ geladenen Strukturen auf der Haaroberfläche wechselwirken.

In der Vergangenheit wurden vielfach Silikon-basierte Verbindungen als Pflegekomponenten in Leave-in-Sprays eingesetzt. Silikone können als Silikonöle vorliegen, die hydrophob sind und mit den lipophilen Komponenten auf der Haaroberfläche wechselwirken und idealerweise einen Schutzfilm auf der Haaroberfläche ausbilden.

Silikonverbindungen können substituiert sein, und zwar mit chemischen Gruppen, die positive Ladungen aufweisen (kationische Silikonverbindungen). Diese Silikonverbindungen können auch mit den negativ geladenen Strukturen an der Haaroberfläche in Wechselwirkung treten.

Neben Silikonverbindungen werden vielfach auch kationische Polymere als pflegende Komponenten eingearbeitet. Hierbei handelt es sich in der Regel um Polymere, die zumeist synthetischen Ursprungs sind. Diese kationische Pflegepolymere können ebenfalls mit den negativ geladenen Oberflächenstrukturen des Haares wechselwirken.

Die Leave-in-Sprays der Vergangenheit hatten somit den Focus, die Haare zu pflegen. Der Einsatz der oben genannten großen Klassen von Pflegekomponenten hat jedoch auch Nachteile. Ein Nachteil, der oft bei Silikon-haltigen Zubereitungen beobachtet wird, ist es, dass die Haare "beschwert" sind, d.h. der Film auf den Haaren wird bei der Haarwäsche nicht vollständig weggewaschen und baut sich immer weiter auf. Eine Frisurgestaltung ist dann überaus schwierig.

Als weiterer Aspekt ist auch das zunehmende Umweltbewusstsein zu nennen. Der Verbraucher wünscht sich Zubereitungen, die natürliche Inhaltsstoffe enthalten und die biologische Abbaubarkeit soll auch gegeben sein. Silikonverbindungen und viele kationische Polymere sind jedoch synthetischer Natur und weisen teilweise eine schlechte biologische Abbaubarkeit auf.

Die Aufgabe der vorliegenden Erfindung bestand nun darin, Leave-in-Sprays für die menschlichen Haare zur Verfügung zu stellen, die eine hinreichende Pflegeleistung erbringen sollten, ohne das Haar dabei zu beschweren. Zudem sollten so weit wie möglich natürliche oder Natur-basierte Verbindungen eingearbeitet werden, ohne dass die Pflegeleistung verringert wird.

Überraschenderweise konnten diese Aufgaben durch ein Leave-in-Spray für die menschlichen Haare enthaltend

- Isoamyllaurat,
- eine Kombination kationischer Tenside, bestehend aus wenigstens einem Esterquat und wenigstens einem Amidoamin und
- optional wenigstens ein weiteres kationisches Tensid ausgewählt aus der Gruppe der Ammoniumsalze ,

gelöst werden.

Das erfindungsgemäße Leave-in-Spray ist eine kosmetische Zubereitung, die für die Anwendung auf menschlichen Haaren, insbesondere Kopfhaaren vorgesehen ist.

Das erfindungsgemäße Leave-in-Spray ist eine wässrige Zubereitung, der Wassergehalt beträgt von 70 bis 95 Gew.-%, bevorzugt von 80 bis 93 Gew.-%, , bezogen auf das Gesamtgewicht des Leave-in-Sprays.

Der Begriff "natürlich" im Zusammenhang mit natürlichen Inhaltsstoffen für Kosmetika ist in der ISO 16128 festgelegt. Gemäß dieser Richtlinie kann man natürliche, kosmetische Inhaltsstoffe zusammenfassend als Stoffe beschreiben, die von Pflanzen, Tieren, Mineralien oder Mikroorganismen erhältlich sind, eingeschlossen sind auch solche Stoffe, die von den genannten Quellen durch physikalische Prozesse, Fermentationsprozesse (nur solche Fermentationsprozesse, die auch in der Natur ablaufen und die zu Produkten führen, die auch in der Natur entstehen) und andere Herstellungsmethoden ohne beabsichtigte chemische Modifikation erhalten werden. Die Mikroorganismen dürfen nur solche sein, die nicht gentechnisch modifiziert wurden.

Im Sinne der vorliegenden Erfindung sind Natur-basierte Substanzen, insbesondere Natur-basierte Polymere, Verbindungen, die von natürlichen Polymeren abgeleitet sind, beispielsweise durch chemische Modifizierungen.

Der Begriff der biologischen Abbaubarkeit beschreibt den Prozess des Abbaus von organischen Verbindungen durch Lebewesen, insbesondere Saprobionten. Im Idealfall entstehen anorganische Stoffe, wie beispielsweise $CO_2$, $O_2$ und Ammoniak; Verbindungen, die von Pflanzen und Mikroorganismen wieder für den Aufbau von organischen Verbindungen genutzt werden.

Organische, chemische Verbindungen, die leicht biologisch abbaubar sind, sind nach OECD 301, für kosmetische Zusammensetzungen meist nach OECD 301 B klassifiziert. Diese Substanzen oder Zusammensetzungen können rasch und vollständig abgebaut werden.

Organische, chemische Verbindungen, die nach OECD 302 klassifiziert sind, sind zwar eingeschränkt, jedoch grundsätzlich biologisch abbaubar.

Isoamyl Laurat ist ein Ester der Fettsäure Laurinsäure und des Alkohols Isoamylalkohol. Laurinsäure kann vorteilhaft aus natürlichen Quellen gewonnen werden, beispielsweise aus den Samen der Früchte von Palmölpflanzen. Isoamylalkohol kann beispielsweise aus Zuckerrohr und dabei insbesondere aus den Fuselölen, einem Nebenprodukt bei der Herstellung von Bioethanol, gewonnen werden. Nach Veresterung der so gewonnen Rohstoffe, wird ein Isoamyl Laurat erhalten, das aus natürlichen Quellen stammt.

Isoamyl Laurat ist an sich bekannt und kann in der Kosmetik eingesetzt werden, um beispielsweise die Haut geschmeidig zu machen. Weiterhin hat Isoamyl Laurat eine emulgierende Wirkung und kann Emulsionen stabilisieren.

In dem erfindungsgemäßen Leave-in-Spray ist Isoamyl Laurat vorteilhaft mit einem Gehalt von 0,05 bis 2,0 Gew.-%, bevorzugt von 0,1 bis 1,0 Gew.-% enthalten, bezogen auf das Gesamtgewicht des Leave-in-Sprays.

Isoamyl Alkohol kann beispielsweise von der Firma Solvay unter dem Handelsnamen Mackaderm Lia MB erworben werden.

Das erfindungsgemäße Leave-in-Spray enthält eine Kombination kationischer Tenside, bestehend aus wenigstens einem Esterquat und wenigstens einem Amidoamin.

Esterquats können als quaternierte Fettsäurealkanolaminestersalze bezeichnet werden. Ausgehend von dieser allgemeinen Beschreibung für Esterquats können verschiedene chemische Strukturformeln für Esterquats angegeben werden.

Im Sinne der vorliegenden Erfindung können dies zu einem Esterquats sein, die bevorzugt aus Esterquats ausgewählt werden, die sich durch folgende Struktur beschreiben lassen:

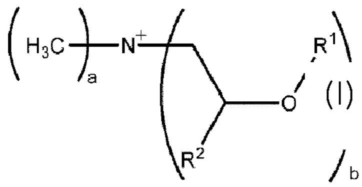

(I)

wobei mit $R^1$ ein Acylrest einer mindestens einfach ungesättigten Fettsäure mit einer Kettenlänge von 18 bis 24 Kohlenstoffatomen oder der Acylrest der Isostearinsäure oder Rizinolsäure, $R^2$ ein Alkylrest mit 1 bis 6 Kohlenstoffatomen, bevorzugt Methyl, Ethyl, Propyl, Isopropyl, besonders bevorzugt Methyl, a = 1 bis 3 und b = 1 bis 3, mit der Maßgabe, dass a + b = 4 sind.

Es ist vorteilhaft, wenn a = 2 und b = 2; weiter vorteilhaft ist es, wenn a = 2, b = 2 und $R^1$ ein Acylrest der Isostearinsäure und ein Acylrest der Ölsäure ist.

Insbesondere vorteilhaft ist es, wenn der Esterquat Bis-(Isostearoyl/Oleyl Isopropyl) Dimonium Methosulfat ist. Bis-(Isostearoyl/Oleyl Isopropyl) Dimonium Methosulfat kann beispielsweise von der Firma Evonik Industries unter der Handelsbezeichnung Varisoft EQ 100 erworben werden.

Zum anderen können die Esterquats bevorzugt aus Esterquats ausgewählt werden, die sich durch folgende Struktur beschreiben lassen:

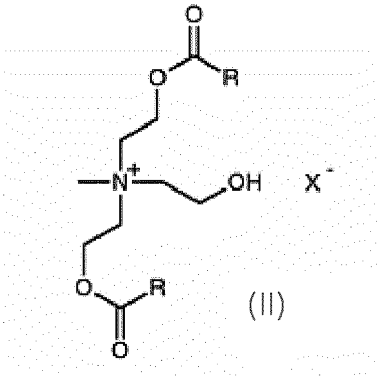

(II)

wobei R einem organischen Rest mit 10 bis 30, bevorzugt 12 bis 24 Kohlenstoffatomen entspricht, insbesondere ein substituierter oder unsubstituierter, verzweigter oder unverzweigter Alkylrest, der auch ungesättigt sein kann, wobei der Alkylrest bevorzugt ein linearer gesättigter Alkylrest mit 14 bis 20 Kohlenstoffatomen ist. Das Gegenion $X^-$ wird bevorzugt aus der Gruppe Chlorid, Bromid und Methosulfat ausgewählt. Ganz besonders bevorzugt sind die Verbindungen Dipalmitoylethyl Hydroxyethylmonium Methosulfate, Dioleylethyl Hydroxyethylmonium Methosulfate und Distearoylethyl Hydroxyethylmonium Methosulfate, wobei Distearoylethyl Hydroxyethylmonium Methosulfate am meisten bevorzugt ist.

Distearoylethyl Hydroxyethylmonium Methosulfate kann beispielsweise als Dehyquart F 75 T bei der Firma BASF bezogen werden.

Insbesondere vorteilhaft ist es, wenn in dem erfindungsgemäßen Leave-in-Spray wenigstens ein Esterquat, beschrieben durch die Strukturformel I und wenigstens ein Esterquat, beschrieben durch die Strukturformel II enthalten ist, weiter insbesondere sind Bis-(Isostearoyl/Oleyl Isopropyl) Dimonium Methosulfat und Distearoylethyl Hydroxyethylmonium Methosulfate enthalten.

Das wenigstens eine Esterquat ist in dem erfindungsgemäßen Leave-in-Spray mit einem Gesamtgehalt von 0,01 bis 10 Gew.-%, bevorzugt 0,1 bis 7,0 Gew.-% und besonders bevorzugt 0,2 bis 5,0 Gew.-% enthalten, bezogen auf das Gesamtgewicht des Leave-in-Sprays und bezogen auf den jeweiligen Aktivgehalt.

Die erfindungsgemäßen Amidoamine sind quaternierten Fettsäureamidoamine und können beispielsweise durch die Umsetzung von Fettsäuren mit mehrwertigen Aminen und nachfolgende Quaternierung der freien Aminfunktion erhalten werden.

Vorteilhafte Verbindungen sind beispielsweise Stearylamidopropyltrimmonium Chloride, Ricinoleamidopropyltrimonnium Chloride und Palmitamidopropyltrimonium Chloride.

Eine erfindungsgemäß bevorzugte Verbindung ist Palmitamidopropyltrimonium Chloride, das beispielsweise unter der Handelsbezeichnung Varisoft PATC von Evonik Industries bezogen werden kann.

Das wenigstens eine Amidoamin wird mit einem Gesamtgehalten von 0,05 Gew.-% bis 5,0 Gew.-%, bevorzugt von 0,01 Gew.-% bis 2,0 Gew.-% , insbesondere bevorzugt von 0,2 Gew.-% bis 1,5 Gew.-% , bezogen auf das Gesamtgewicht des Leave-in-Sprays und den Aktivgehalt, eingesetzt.

Zusätzlich kann wenigstens ein weiteres kationisches Tensid in dem erfindungsgemäßen Leave-in-Spray enthalten sein. Das weitere kationische Tensid ist kein Esterquat und kein Amidoamin. Es ist bevorzugt, wenn das wenigstens eine weitere kationische Tensid aus der Gruppe der Ammoniumsalze gewählt wird. Weiter bevorzugt ist es, wenn das wenigstens eine weitere kationische Tensid aus der Gruppe der primären Ammoniumsalze gewählt wird, die nur einen langkettigen organischen Rest aufweisen. Besonders bevorzugt ist es, wenn das das wenigstens eine weitere kationische Tensid ausgewählt wird aus der Gruppe Cetyltrimethylammoniumchlorid (Cetrimonium Chlorid), Stearyltrimethylammoniumchlorid und/oder Behentrimoniumchlorid. Am meisten bevorzugt ist es, wenn das wenigstens eine weitere kationische Tensid Behentrimoniumchlorid ist.

Wenn in dem erfindungsgemäßen Leave-in-Spray wenigstens ein weiteres kationisches Tensid enthalten ist, so liegt dieses Tensid mit einem Gesamtgehalt von 0,1 bis 5,0 Gew.-%, bevorzugt 0,5 bis 3,0 Gew.-% vor, bezogen auf das Gesamtgewicht des Leave-in-Sprays und bezogen auf den Aktivgehalt. Im Sinne der vorliegenden Erfindung ist der

Gesamtgehalt des wenigstens einen weiteren kationischen Tensids nicht im Gesamtgehalt der kationischen Tenside ausgewählt aus der Gruppe der Esterquats und Amidoamine enthalten.

Die erfindungsgemäße Zubereitung liegt vorteilhaft in Form einer Emulsion vor, insbesondere in Form einer O/W-Emulsion.

Im Sinne der vorliegenden Erfindung kann das Leave-in-Spray ein-phasig oder zwei-phasig vorliegen. Auch Übergänge sind möglich, d.h. das Leave-in-Spray liegt nach der Herstellung oder nach dem Schütteln ein-phasig vor und eine Phasentrennung tritt 1 bis 4 Stunden später auf. In diesem Fall und bei zwei-phasigen Leave-in-Sprays ist es erforderlich, die Zubereitung vor dem Gebrauch zu schütteln, damit eine weitgehend homogene Zubereitung auf das Haar aufgetragen werden kann.

Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert. Die Flüssigkeiten (rein oder als Lösungen) liegen in einer Emulsion in einer mehr oder weniger feinen Verteilung vor, die im Allgemeinen nur begrenzt stabil ist.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter, zum Beispiel elektrische Leitfähigkeit, Sensorik, Anfärbbarkeit der kontinuierlichen Phase, einer O/W-Emulsion ist durch das Wasser geprägt. Bei einer Wasser-in-Öl-Emulsion (W/O-Emulsion, z.B. Butter) handelt es sich um das umgekehrte Prinzip, wobei der Grundcharakter hier durch das Öl bestimmt wird.

Emulsionen benötigen zu ihrer Bildung und zur Stabilisierung im Allgemeinen einen oder mehrere Emulgatoren, um über einen kosmetisch akzeptablen Zeitraum stabil zu sein.

Emulgatoren bewirken, dass die zwei nicht miteinander mischbaren Flüssigkeiten (zum Beispiel Öl in Wasser) sich zu einer Emulsion vermengen lassen. Aufgrund des amphiphilen Charakters dringen sie mit ihrem fettlöslichen Teil in das Öl ein. Durch den hydrophilen Teil können die durch Rühren oder Homogenisieren entstandenen Öltröpfchen in der wässrigen Umgebung dispergiert werden. Emulgatoren haben primär keinen waschaktiven, tensidischen Charakter.

Emulgatoren setzen die Grenzflächenspannung zwischen den beiden Phasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.

Damit Verbindungen als Emulgatoren wirksam sein können, müssen sie eine bestimmte Molekülstruktur aufweisen. Strukturelles Kennzeichen solcher Verbindungen ist ihr amphiphiler Molekülaufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe). Dabei kann zwischen nicht-ionischen, anionischen und kationischen Emulgatoren unterschieden werden.

Ein Kennzeichen für die Hydrophilie eines gegebenen Emulgators ist dessen HLB-Wert, der mithilfe nachfolgender Formel bestimmt werden kann:

$$HLB = 20 \times (1 - M_{lipophil}/M),$$

wobei $M_{lipophil}$ für die Molmasse des lipophilen Anteils im Emulgator und
M für die Molmasse des gesamten Emulgators steht.

Im Allgemeinen gelten Emulgatoren mit einem HLB-Wert bis ca. 8 als W/O-Emulgatoren. O/W-Emulgatoren hingegen weisen HLB-Werte von größer 8 bis etwa 15 auf. Substanzen mit HLB-Werten größer 15 werden häufig als Lösungsvermittler bezeichnet.

Es ist vorteilhaft hydrophile Emulgatoren ausgewählt, also solche, die einen HLB-Wert von $\geq 9{,}5$ haben. Weiter vorteilhaft werden die Emulgatoren Ceteareth-20, Polysorbate 60, Polysorbat 20, und/oder Laureth-4 ausgewählt.

Wenn in dem erfindungsgemäßen Leave-in-Spray wenigstens ein Emulgator enthalten ist, so liegt der wenigstens eine Emulgator mit einem Gesamtgehalt von 0,15 bis 3,0 Gew.-%, bevorzugt 0,3 bis 2,0 Gew.-% vor, bezogen auf das Gesamtgewicht des Leave-in-Sprays. Im Sinne der vorliegenden Erfindung ist der Gehalt von Isoamyl Laurat nicht im Gesamtgehalt des wenigstens einen Emulgators enthalten.

In dem erfindungsgemäßen Leave-in-Spray kann zusätzlich wenigstens ein kationisches Polymer enthalten sein. Dieses kationische Polymer ist ein Natur-basiertes Polymer; das bedeutet, dass es sich um ein natürliches Polymer handelt, das eine chemische Modifizierung erfahren hat, um Ammoniumgruppen in das Molekül einzuführen. Derartige Moleküle weisen eine deutlich bessere biologische Abbaubarkeit auf als kationische Polymere, die rein synthetischer Natur sind.

Diese kationischen, Natur-basierten Polymere können aus quaternisierten Cellulose-Derivaten, wie beispielsweise Polyquaternium 10, oder kationischen Guar-Derivaten, wie beispielsweise Guar Hydroxypropyltrimonium Chloride,

ausgewählt werden. Es ist bevorzugt, wenn Guar Hydroxypropyltrimonium Chloride in dem erfindungsgemäßen Leave-in-Spray enthalten ist. Guar Hydroxypropyltrimonium Chloride kann beispielsweise unter der Handelsbezeichnung Dehyquart Guar TC bei der Firma BASF bezogen werden.

Das wenigstens eine kationische, Natur-basierte Polymer liegt in dem erfindungsgemäßen Leave-in-Spray mit einem Gesamtgehalt von 0,005 bis 1,0 Gew.-%, bevorzugt 0,02 bis 0,75 Gew.-% vor, bezogen auf das Gesamtgewicht des Leave-in-Sprays und bezogen auf den Aktivgehalt.

In dem erfindungsgemäßen Leave-in-Spray kann zusätzlich wenigstens ein Moisturizer enthalten sein. Als Moisturizer werden Stoffe oder Stoffgemische bezeichnet, welche kosmetischen Zubereitungen die Eigenschaft verleihen, nach dem Auftragen bzw. Verteilen auf der Haaroberfläche die Feuchtigkeitsabgabe zu reduzieren und/oder die Hydratation der Haaroberfläche bzw. der tiefer liegenden Schichten positiv zu beeinflussen. Vorteilhafte Moisturizer im Sinne der vorliegenden Erfindung sind beispielsweise Glycerin, Sorbitol, Butylenglykol, Propylenglykol, Milchsäure und/oder Lactate, insbesondere Natriumlactat, Biosaccaride Gum-1, Glycine Soja, Ethylhexyloxyglycerin, Pyrrolidoncarbonsäure und Harnstoff. Bevorzugt wird der wenigstens eine Moisturizer aus der Gruppe Glycerin, Sorbitol, Butylenglykol, Propylenglykol, Milchsäure und/oder Salze der Milchsäure ausgewählt. Es können auch zwei oder mehr verschiedene Moisturizer enthalten sind, beispielsweise Milchsäure und Glycerin.

Wenn in dem erfindungsgemäßen Leave-in-Spray wenigstens ein Moisturizer enthalten ist, so liegt er mit einem Gesamtgehalt von 0,1 bis 10 Gew.-%, bevorzugt 0,5 bis 5,0 Gew.-%, bezogen auf das Gesamtgewicht des Leave-in-Sprays, vor.

In dem erfindungsgemäßen Leave-in-Spray kann zusätzlich wenigstens eine Substanz der Vitamine der B Gruppe und/oder des Vitamin B-Komplexes enthalten sein. Substanzen der Vitamin B Gruppe und/oder des Vitamin B-Komplexes sind üblicherweise wasserlöslich und spielen insbesondere für den Zellmetabolismus bei Pflanzen und Tieren eine besondere Rolle.

Beispiele erfindungsgemäßer Substanzen der Vitamine der B Gruppe und/oder des Vitamin B-Komplex sind unter anderem Thiamin (Vitamin B1), Riboflavin (Vitamin B2), Nicotinsäure (Vitamin B3), Nicotinsäureamid (Niacinamid), Pantothensäure (Vitamin B5), Panthenol (Provitamin B5), Panthenoltriacetat, Panthenolmonoethylether, Pantolacton, Pyridoxin und Pyridoxal.

Bevorzugt ist die Verwendung von Panthenol als Substanz der Vitamine der B Gruppe und/oder des Vitamin B-Komplexes in dem erfindungsgemäßen Leave-in-Spray.

Panthenol kann beispielsweise unter dem Handelsnamen D-Panthenol 75 W als 77 % Lösung in Wasser von der BASF bezogen werden.

Wenn in dem erfindungsgemäßen Leave-in-Spray wenigstens eine Substanz der Vitamine der B Gruppe und/oder des Vitamin B-Komplex enthalten ist, so liegt diese wenigstens eine Substanz mit einem Gesamtgehalt von 0,005 Gew.-% bis 5 Gew.-%, bevorzugt 0,05 Gew.-% bis 2 Gew.-%, insbesondere bevorzugt 0,01 Gew.-% bis 1,0 Gew.-%, bezogen auf das Gesamtgewicht des Leave-in-Sprays und den Aktivgehalt, vor.

In dem erfindungsgemäßen Leave-in-Spray kann vorteilhaft wenigstens ein Konservierungsmittel enthalten sein. Konservierungsmittel sind diejenigen konservierenden Substanzen, die gemäß Kosmetikverordnung für Deutschland und gemäß der Verordnung (EG) Nr. 1223/2009 über kosmetische Mittel für den Einsatz in kosmetischen Produkten für Europa zugelassen sind.

Als Konservierungsmittel können weiter vorteilhaft Phenoxyethanol, Methyl-, Ethyl-, Propylparaben, Benzylalkohol, Sorbinsäure und/oder Salze der Sorbinsäure, beispielsweise Kalium Sorbat, Benzoesäure und/oder Salze der Benzoesäure, beispielsweise Natrium Benzoat, Salicylsäure und/oder Salze der Salicylsäure, beispielsweise Natrium Salicylat eingesetzt werden. Es ist insbesondere vorteilhaft Phenoxyethanol einzusetzen.

Wenn in dem erfindungsgemäßen Leave-in-Spray wenigstens ein Konservierungsmittel enthalten ist, so liegt das wenigstens eine Konservierungsmittel mit einem Gesamtgehalt von 0,0001 bis 8,0 Gew.-%, bevorzugt von 0,0005 bis 4,0 Gew.-%, besonders bevorzugt von 0,001 bis 2,0 Gew.-% in der erfindungsgemäßen Zubereitung vor, bezogen auf das Gesamtgewicht der Zubereitung.

Weiterhin ist es vorteilhaft, wenn die Zubereitung ein Parfüm enthält. Parfüme sind Mischungen aus Parfümrohstoffen. Das Parfüm liegt vorteilhaft mit einem Gehalt von 0.05 bis 2,0 Gew.-% vor, bezogen auf das Gesamtgewicht der Zubereitung.

Die erfindungsgemäße Zubereitung kann auf jede, für eine derartige Zubereitung im StdT bekannte und geeignete, Weise hergestellt werden.

Dem erfindungsgemäßen Leave-in-Spray werden keine Silikon-Verbindungen zugesetzt, ebenso sind keine Silikon-Verbindungen enthalten, die mit an sich anderen Rohstoffen in die Zubereitung gelangen. Das erfindungsgemäße Leave-in-Spray ist also frei von Silikon-Verbindungen. Silikon-Verbindungen zeichnen sich durch das Vorhandensein von Silicium- und Sauerstoffatomen aus, die miteinander verknüpft sind und eine Vielzahl verschiedener Verbindungen ausbilden können.

Weiterhin werden dem erfindungsgemäßen Leave-in-Spray keine PEG-haltigen Verbindungen zugesetzt, ebenso sind keine PEG-haltigen Verbindungen enthalten, die mit an sich anderen Rohstoffen in die Zubereitung gelangen. Das erfindungsgemäße Leave-in-Spray ist also frei von PEG-haltigen-Verbindungen. PEG ist die Abkürzung für Polyethylenglycol; viele der Struktureinheiten (-$CH_2$-$CH_2$-O-) sind dabei miteinander verknüpft. Diese Strukturen können bewirken, dass eine Penetration durch die Haut bewirkt oder verstärkt wird. Unter PEG-haltige Verbindungen werden Polyethylenglykole und Polyethylenglykolderivate verstanden.

Ebenso werden den Leave-in-Sprays keine synthetischen Polyquaternium-Verbindungen zugesetzt, auch sind keine synthetischen Polyquaternium-Verbindungen enthalten, die mit an sich anderen Rohstoffen in die Zubereitung gelangen. Polyquaternium-Verbindungen sind polymere Verbindungen, die sich aus einem oder verschiedenen Monomeren zusammensetzen. Diese Monomere können rein synthetischer Natur sein. Ebenso kann das gesamte Polymer synthetischer Natur sein. Derartige Polymere werden nicht von lebenden Organismen in biologischen Prozessen hergestellt. Die chemische Struktur der Polyquaternium-Verbindungen ist verschieden; das gemeinsame Merkmal ist jedoch das Vorhandensein vom Ammonium-Gruppen. Das erfindungsgemäße Leave-in-Spray ist frei von synthetischen Polyquaternium-Verbindungen.

Um die Pflegeleistung zu untersuchen wurde in einem *in-vitro*-Versuch die Naßkämmkraft in Bezug auf die Anwendung einer ausgewählten erfindungsgemäßen Zubereitung bestimmt. Dazu wurde ein Produkt gemäß Ausführungsbeispiel 1 auf jeweils 7 Haarsträhnen (Haarsträhne von je 2g, geschädigt) aufgetragen. Zuvor wurden die Haarsträhnen mit einem Testshampoo gewaschen und das erste Mal in Bezug auf die Naßkämmbarkeit und die dabei einzusetzende Kraft vermessen (Zeitpunkt to). Dann wurde die Messung nach Anwendung des erfindungsgemäßen Produktes wiederholt (Zeitpunkt ti). Die Ergebnisse sind in Abbildungen 1a und 1b dargestellt. In Abbildung 1a ist auf der y-Achse die mittlere Kraft aufgetragen, in Abbildung 1b die maximale Kraft. Aus beiden Abbildungen lässt sich ablesen, dass nach Verwendung des erfindungsgemäßen Produktes deutlich weniger Kraft für das Kämmen nötig ist. Wenn weniger Kraft nötig ist, ist das Kämmen einfacher, d.h. die Oberfläche des Haares ist glatter. Dies ist ein Beleg für eine gute Pflegeleistung des erfindungsgemäßen Produktes.

Weiterhin wurde eine Home-in-Use Studie durchgeführt. 83 weiblichen Anwenderinnen im Alter von 18 bis 65 Jahren wurde ein erfindungsgemäßes Produkt (Produkt gemäß Ausführungsbeispiel 1) zur Verfügung gestellt. Dieses Produkt sollte 3 bis 4mal pro Woche für einen Zeitraum von 10 Tagen im nassen und trockenen Haar angewendet werden. Mithilfe eines Fragebogens, der überwiegend Aussagen in Bezug auf die Haareigenschaften nach der Anwendung enthielt, wurde die Akzeptanz des Produktes evaluiert und Erkenntnisse darüber erhalten, welche Haareigenschaften nach der Anwendung besonders gut bewertet wurden. Eine besonders gute Bewertung zeigt an, dass diese Eigenschaften von den Anwenderinnen wahrgenommen werden und dass somit das Produkt diese Eigenschaften auf dem Haar hervorrufen kann. Besonders gut bewertet wurde u.a. der Haarglanz; 98 % der Anwenderinnen attestierten dem Haar einen natürlichen Glanz nach der Anwendung des erfindungsgemäßen Produktes, 92 % einen langanhaltenden Glanz. 93 % der Anwenderinnen bestätigten, dass sich das Haar nach Anwendung des erfindungsgemäßen Produktes besser kämmen lässt, 95 %, dass sich das nasse Haar besser entwirren lässt und 86 %, dass sich das trockene Haar besser entwirren lässt. 98 % der Anwenderinnen beurteilten ihr Haar nach der Anwendung des erfindungsgemäßen Produktes als gut gepflegt und sogar 99 % der Anwenderinnen bestätigten, dass das erfindungsgemäße Produkt das Haar mild pflegt. 88 % der Anwenderinnen bestätigten, dass das erfindungsgemäße Produkt das Haar nicht beschwert und sogar 94 %, empfanden, dass sich nach der Anwendung des erfindungsgemäßen Produktes das Haar leicht und gepflegt anfühlt. Diese Beurteilungen zeigen, dass dem erfindungsgemäße Leave-in-Spray eine pflegende Wirkung, die Verbesserung des Haarglanzes und keine Beschwerung des Haares attestiert wird und somit die Aufgaben, die der vorliegenden Erfindung zugrunde liegen, gelöst sind.

Zusammenfassend ist die vorliegende Patentanmeldung auf den Gegenstand gerichtet, der in den folgenden, nummerierten Paragrafen beschrieben ist:

1. Leave-in-Spray, vorgesehen für die kosmetische Anwendung auf menschlichen Haaren, enthaltend

- Isoamyllaurat,
- eine Kombination kationischer Tenside, bestehend aus wenigstens einem Esterquat und wenigstens einem Amidoamin und
- optional wenigstens ein weiteres kationisches Tensid ausgewählt aus der Gruppe der Ammoniumsalze.

2. Leave-in-Spray gemäß Paragraf 1 dadurch charakterisiert, dass der Wassergehalt von 70 bis 95 Gew.-%, bevorzugt von 80 bis 93 Gew.-%, beträgt, bezogen auf das Gesamtgewicht des Leave-in-Sprays.

3. Leave-in-Spray gemäß Paragraf 1 und/oder 2 dadurch charakterisiert, dass Isoamyl Laurat mit einem Gehalt von 0,05 bis 2,0 Gew.-%, bevorzugt von 0,1 bis 1,0 Gew.-% enthalten ist, bezogen auf das Gesamtgewicht des Leave-in-Sprays.

4. Leave-in-Spray gemäß wenigstens einem der vorstehenden Paragrafen dadurch charakterisiert, dass der wenigstens eine Esterquat ausgewählt wird aus Esterquats, die durch folgende Struktur beschrieben werden:

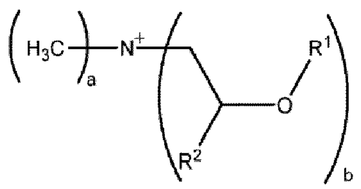

(I)

wobei mit $R^1$ ein Acylrest einer mindestens einfach ungesättigten Fettsäure mit einer Kettenlänge von 18 bis 24 Kohlenstoffatomen oder der Acylrest der Isostearinsäure oder Rizinolsäure, $R^2$ ein Alkylrest mit 1 bis 6 Kohlenstoffatomen, bevorzugt Methyl, Ethyl, Propyl, Isopropyl, besonders bevorzugt Methyl, a = 1 bis 3 und b = 1 bis 3, mit der Maßgabe, dass a + b = 4 sind, weiter bevorzugt ist a = 2 und b = 2; noch weiter bevorzugt ist es, wenn a = 2, b = 2 und $R^1$ ein Acylrest der Isostearinsäure und ein Acylrest der Ölsäure ist, insbesondere bevorzugt ist der Esterquat Bis-(Isostearoyl/Oleyl Isopropyl) Dimonium Methosulfat.

5. Leave-in-Spray gemäß wenigstens einem der vorstehenden Paragrafen dadurch charakterisiert, dass der wenigstens eine Esterquat ausgewählt wird aus Esterquats, die durch die folgende Struktur beschrieben werden:

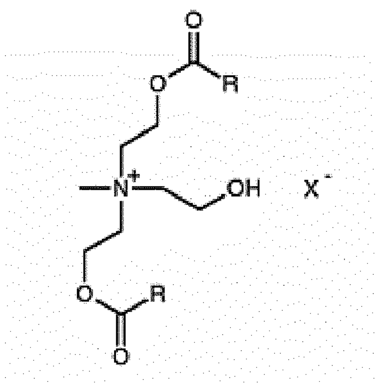

(II)

wobei R ein substituierter oder unsubstituierter, verzweigter oder unverzweigter und/oder gesättigter oder ungesättigter Alkylrest mit 14 bis 20 Kohlenstoffatomen, bevorzugt ein linearer, gesättigter Alkylrest mit 14 bis 20 Kohlenstoffatomen.

6. Leave-in-Spray gemäß Paragraf 5 dadurch charakterisiert, dass der wenigstens eine Esterquat ausgewählt wird aus den Verbindungen Dipalmitoylethyl Hydroxyethylmonium Methosulfate, Dioleylethyl Hydroxyethylmonium Methosulfate und/oder Distearoylethyl Hydroxyethylmonium Methosulfate, bevorzugt ist der Esterquat Distearoylethyl Hydroxyethylmonium Methosulfate.

7. Leave-in-Spray gemäß wenigstens einem der vorstehenden Paragrafen dadurch charakterisiert, dass wenigstens ein Esterquat, beschrieben durch die Strukturformel I und wenigstens ein Esterquat, beschrieben durch die Strukturformel II enthalten ist, bevorzugt sind Bis-(Isostearoyl/Oleyl Isopropyl) Dimonium Methosulfat und/oder Distearoylethyl Hydroxyethylmonium Methosulfate enthalten.

8. Leave-in-Spray gemäß wenigstens einem der vorstehenden Paragrafen dadurch charakterisiert, dass der wenigstens eine Esterquat mit einem Gesamtgehalt von 0,01 bis 10 Gew.-%, bevorzugt 0,1 bis 7,0 Gew.-% und besonders bevorzugt 0,2 bis 5,0 Gew.-% enthalten ist, bezogen auf das Gesamtgewicht des Leave-in-Sprays und bezogen auf den jeweiligen Aktivgehalt.

9. Leave-in-Spray gemäß wenigstens einem der vorstehenden Paragrafen dadurch charakterisiert, dass das wenigstens eine Amidoamin ausgewählt wird aus Stearylamidopropyltrimmonium Chloride, Ricinoleamidopropyltrimonnium Chloride und/oder Palmitamidopropyltrimonium Chloride, bevorzugt ist das wenigstens eine Amidoamin Palmitamidopropyltrimonium Chloride.

10. Leave-in-Spray gemäß wenigstens einem der vorstehenden Paragrafen dadurch charakterisiert, dass das wenigstens eine Amidoamin mit einem Gesamtgehalten von 0,05 Gew.-% bis 5,0 Gew.-%, bevorzugt von 0,1 Gew.-% bis 2,0 Gew.-%, insbesondere bevorzugt von 0,2 Gew.-% bis 1,5 Gew.-% enthalten ist, bezogen auf das Gesamtgewicht des Leave-in-Sprays und den Aktivgehalt, eingesetzt.

11. Leave-in-Spray gemäß wenigstens einem der vorstehenden Paragrafen dadurch charakterisiert, dass zusätzlich wenigstens ein weiteres kationisches Tensid, das kein Esterquat und kein Amidoamin ist, enthalten ist, bevorzugt ausgewählt aus der Gruppe der Ammoniumsalze, weiter bevorzugt aus der Gruppe der primären Ammoniumsalze, die nur einen langkettigen organischen Rest aufweisen, weiter bevorzugt ausgewählt aus der Gruppe Cetyltrimethylammoniumchlorid (Cetrimonium Chlorid), Stearyltrimethylammoniumchlorid und/oder Behentrimoniumchlorid.

12. Leave-in-Spray gemäß wenigstens einem der vorstehenden Paragrafen dadurch charakterisiert, dass das wenigstens eine weitere kationische Tensid mit einem Gesamtgehalt von 0,1 bis 5,0 Gew.-%, bevorzugt 0,5 bis 3,0 Gew.-% enthalten ist, bezogen auf das Gesamtgewicht des Leave-in-Sprays und bezogen auf den Aktivgehalt.

13. Leave-in-Spray gemäß wenigstens einem der vorstehenden Paragrafen dadurch charakterisiert, dass das Leave-in-Spray in Form einer Emulsion vorliegt, bevorzugt in Form einer O/W-Emulsion.

14. Leave-in-Spray gemäß wenigstens einem der vorstehenden Paragrafen dadurch charakterisiert, dass zusätzlich hydrophile Emulgatoren enthalten sind, bevorzugt solche mit einem HLB-Wert von $\geq 9{,}5$, weiter bevorzugt ausgewählt aus Ceteareth-20, Polysorbate 60, Polysorbat 20 und/oder Laureth-4.

15. Leave-in-Spray gemäß wenigstens einem der vorstehenden Paragrafen dadurch charakterisiert, dass der wenigstens eine Emulgator mit einem Gesamtgehalt von 0,15 bis 3,0 Gew.-%, bevorzugt 0,3 bis 2,0 Gew.-% vorliegt, bezogen auf das Gesamtgewicht des Leave-in-Sprays.

16. Leave-in-Spray gemäß wenigstens einem der vorstehenden Paragrafen dadurch charakterisiert, dass zusätzlich wenigstens ein kationisches, Natur-basiertes Polymer enthalten ist, bevorzugt ausgewählt aus quaternisierten Cellulose-Derivaten und/oder kationischen Guar-Derivaten, insbesondere bevorzugt Guar Hydroxypropyltrimonium Chloride.

17. Leave-in-Spray gemäß wenigstens einem der vorstehenden Paragrafen dadurch charakterisiert, dass das wenigstens eine kationische, Natur-basierte Polymer liegt mit einem Gesamtgehalt von 0,005 bis 1,0 Gew.-%, bevorzugt 0,02 bis 0,75 Gew.-% vor, bezogen auf das Gesamtgewicht des Leave-in-Sprays und bezogen auf den Aktivgehalt.

18. Leave-in-Spray gemäß wenigstens einem der vorstehenden Paragrafen dadurch charakterisiert, dass das Leave-in-Spray frei von Silikon-Verbindungen ist.

19. Leave-in-Spray gemäß wenigstens einem der vorstehenden Paragrafen dadurch charakterisiert, dass das Leave-in-Spray frei von PEG-haltigen-Verbindungen ist.

20. Leave-in-Spray gemäß wenigstens einem der vorstehenden Paragrafen dadurch charakterisiert, dass das Leave-in-Spray frei vom synthetischen Polyquaternium-Verbindungen.

**Beispiele:**

Die untenstehenden Beispiele sollen die Erfindung illustrieren und nicht einschränken. Die Angaben beziehen sich auf Gew.-% und den jeweiligen Aktivgehalt, soweit nicht anders angegeben.

| INCI Bezeichnung | Bsp. 1 | Bsp. 2 | Bsp. 3 | Bsp. 4 | Bsp. 5 | Bsp. 6 |
|---|---|---|---|---|---|---|
| Ceteareth-20 | 1 | | | 0,5 | 1 | |
| Polysorbat 20 | | 0,8 | | 0,3 | | 0,4 |
| Polysorbat 60 | | | 1,5 | | | 0,4 |
| Laureth-4 | | | | | | |
| Isoamyl Laurate | 0,25 | 0,3 | 0,25 | 0,5 | 0,1 | 0,3 |

(fortgesetzt)

| INCI Bezeichnung | Bsp. 1 | Bsp. 2 | Bsp. 3 | Bsp. 4 | Bsp. 5 | Bsp. 6 |
|---|---|---|---|---|---|---|
| Distearoylethyl Hydroxyethylmonium Methosulfate | 1,4 | 1 | 1 | 2 | 1 | 1,5 |
| Behentrimonium Chloride | 1,4 | 1,75 | 1 | 2 | 1,75 | 1,4 |
| Bis-(Isostearoyl/Oleoyl Isopropyl) Dimonium Methosulfate | 2 | 2,5 | 3 | 1,5 | 2,5 | 2 |
| Guar Hydroxypropyltrimonium Chloride | 0,09 | 0,1125 | 0,135 | 0,09 | 0,18 | 0,27 |
| Palmitamidopropyltrimonium Chloride | 0,6 | 1 | 0,8 | 1,25 | 1 | 0,6 |
| Perfume | 1,5 | 1 | 1,2 | 1,5 | 1 | 1 |
| Propylene Glycol | | 1 | | 0,5 | 1 | |
| Phenoxyethanol | 0,6 | | 0,6 | 0,6 | 0,6 | |
| Sodium Benzoate | | 0,5 | | | | 0,5 |
| Aqua | Add 100 | Add 100 | Add 100 | Add 100 | Add 100 | Add 100 |
| Sodium Hydroxide | Adj. | Adj. | Adj. | Adj. | Adj. | Adj. |
| Citric Acid | Adj. | Adj. | Adj. | Adj. | Adj. | Adj. |
| Adj: Erforderliche Menge, um den gewünschten pH-Wert einzustellen. | | | | | | |

**Patentansprüche**

1. Leave-in-Spray, vorgesehen für die kosmetische Anwendung auf menschlichen Haaren, enthaltend

   a. Isoamyllaurat,
   b. eine Kombination kationischer Tenside, bestehend aus wenigstens einem Esterquat und wenigstens einem Amidoamin und
   c. optional wenigstens ein weiteres kationisches Tensid ausgewählt aus der Gruppe der Ammoniumsalze.

2. Leave-in-Spray gemäß Anspruch 1 dadurch charakterisiert, dass der Wassergehalt von 70 bis 95 Gew.-%, bevorzugt von 80 bis 93 Gew.-%, beträgt, bezogen auf das Gesamtgewicht des Leave-in-Sprays.

3. Leave-in-Spray gemäß Anspruch 1 und/oder 2 dadurch charakterisiert, dass Isoamyl Laurat mit einem Gehalt von0,05 bis 2,0 Gew.-%, bevorzugt von 0,1 bis 1,0 Gew.-% enthalten ist, bezogen auf das Gesamtgewicht des Leave-in-Sprays.

4. Leave-in-Spray gemäß wenigstens einem der vorstehenden Ansprüche dadurch charakterisiert, dass der wenigstens eine Esterquat ausgewählt wird aus Esterquats, die durch folgende Struktur beschrieben werden:

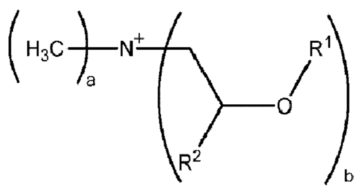

(I)

wobei mit $R^1$ ein Acylrest einer mindestens einfach ungesättigten Fettsäure mit einer Kettenlänge von 18 bis 24 Kohlenstoffatomen oder der Acylrest der Isostearinsäure oder Rizinolsäure, $R^2$ ein Alkylrest mit 1 bis 6 Kohlenstoffatomen, bevorzugt Methyl, Ethyl, Propyl, Isopropyl, besonders bevorzugt Methyl, a = 1 bis 3 und b = 1 bis 3, mit der Maßgabe, dass a + b =4 sind, weiter bevorzugt ist a = 2 und b = 2; noch weiter bevorzugt ist es, wenn a = 2, b = 2 und $R^1$ ein Acylrest der Isostearinsäure und ein Acylrest der Ölsäure ist, insbesondere bevorzugt ist der Esterquat Bis-(Isostearoyl/Oleyl Isopropyl) Dimonium Methosulfat.

5. Leave-in-Spray gemäß wenigstens einem der vorstehenden Ansprüche dadurch charakterisiert, dass der wenigstens eine Esterquat ausgewählt wird aus Esterquats, die durch die folgende Struktur beschrieben werden:

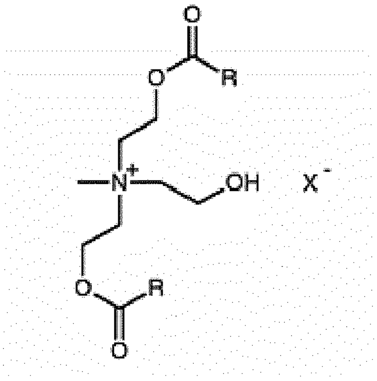

(II)

wobei R ein substituierter oder unsubstituierter, verzweigter oder unverzweigter und/oder gesättigter oder ungesättigter Alkylrest mit 14 bis 20 Kohlenstoffatomen, bevorzugt ein linearer, gesättigter Alkylrest mit 14 bis 20 Kohlenstoffatomen.

6. Leave-in-Spray gemäß Anspruch 5 dadurch charakterisiert, dass der wenigstens eine Esterquat ausgewählt wird aus den Verbindungen Dipalmitoylethyl Hydroxyethylmonium Methosulfate, Dioleylethyl Hydroxyethylmonium Methosulfate und/oder Distearoylethyl Hydroxyethylmonium Methosulfate, bevorzugt ist der Esterquat Distearoylethyl Hydroxyethylmonium Methosulfate.

7. Leave-in-Spray gemäß wenigstens einem der vorstehenden Ansprüche dadurch charakterisiert, dass wenigstens ein Esterquat, beschrieben durch die Strukturformel I und wenigstens ein Esterquat, beschrieben durch die Strukturformel II enthalten ist, bevorzugt sind Bis-(Isostearoyl/Oleyl Isopropyl) Dimonium Methosulfat und Distearoylethyl Hydroxyethylmonium Methosulfate enthalten.

8. Leave-in-Spray gemäß wenigstens einem der vorstehenden Ansprüche dadurch charakterisiert, dass der wenigstens eine Esterquat ausgewählt wird aus mit einem Gesamtgehalt von 0,01 bis 10 Gew.-%, bevorzugt 0,1 bis 7,0 Gew.-% und besonders bevorzugt 0,2 bis 5,0 Gew.-% enthalten ist, bezogen auf das Gesamtgewicht des Leave-in-Sprays und bezogen auf den jeweiligen Aktivgehalt.

9. Leave-in-Spray gemäß wenigstens einem der vorstehenden Ansprüche dadurch charakterisiert, dass das wenigstens eine Amidoamin ausgewählt wird aus Stearylamidopropyltrimmonium Chloride, Ricinoleamidopropyltrimonnium Chloride und/oder Palmitamidopropyltrimonium Chloride, bevorzugt ist das wenigstens eine Amidoamin Palmitamidopropyltrimonium Chloride.

10. Leave-in-Spray gemäß wenigstens einem der vorstehenden Ansprüche dadurch charakterisiert, dass das wenigstens eine Amidoamin mit einem Gesamtgehalten von 0,05 Gew.-% bis 5,0 Gew.-%, bevorzugt von 0,1 Gew.-% bis 2,0 Gew.-%, insbesondere bevorzugt von 0,2 Gew.-% bis 1,0 Gew.-% enthalten ist, bezogen auf das Gesamtgewicht des Leave-in-Sprays und den Aktivgehalt, eingesetzt.

11. Leave-in-Spray gemäß wenigstens einem der vorstehenden Ansprüche dadurch charakterisiert, dass zusätzlich wenigstens ein weiteres kationisches Tensid, das kein Esterquat und kein Amidoamin ist, enthalten ist, bevorzugt ausgewählt aus der Gruppe der Ammoniumsalze, weiter bevorzugt aus der Gruppe der primären Ammoniumsalze, die nur einen langkettigen organischen Rest aufweisen, weiter bevorzugt ausgewählt aus der Gruppe Cetyltrimethylammoniumchlorid (Cetrimonium Chlorid), Stearyltrimethylammoniumchlorid und/oder Behentrimoniumchlorid.

12. Leave-in-Spray gemäß wenigstens einem der vorstehenden Ansprüche dadurch charakterisiert, dass das wenigstens eine weitere kationische Tensid mit einem Gesamtgehalt von 0,1 bis 5,0 Gew.-%, bevorzugt 0,5 bis 3,0 Gew.-% enthalten ist, bezogen auf das Gesamtgewicht des Leave-in-Sprays und bezogen auf den Aktivgehalt.

13. Leave-in-Spray gemäß wenigstens einem der vorstehenden Ansprüche dadurch charakterisiert, dass das Leave-in-Spray in Form einer Emulsion vorliegt, bevorzugt in Form einer O/W-Emulsion.

**14.** Leave-in-Spray gemäß wenigstens einem der vorstehenden Ansprüche dadurch charakterisiert, dass zusätzlich wenigstens ein kationisches, Natur-basiertes Polymer enthalten ist, bevorzugt ausgewählt aus quaternisierten Cellulose-Derivaten und/oder kationischen Guar-Derivaten, insbesondere bevorzugt Guar Hydroxypropyltrimonium Chloride.

**15.** Leave-in-Spray gemäß wenigstens einem der vorstehenden Ansprüche dadurch charakterisiert, dass das wenigstens eine kationische, Natur-basierte Polymer liegt mit einem Gesamtgehalt von 0,005 bis 1,0 Gew.-%, bevorzugt 0,02 bis 0,75 Gew.-% vor, bezogen auf das Gesamtgewicht des Leave-in-Sprays und bezogen auf den Aktivgehalt.

Abbildung 1a:

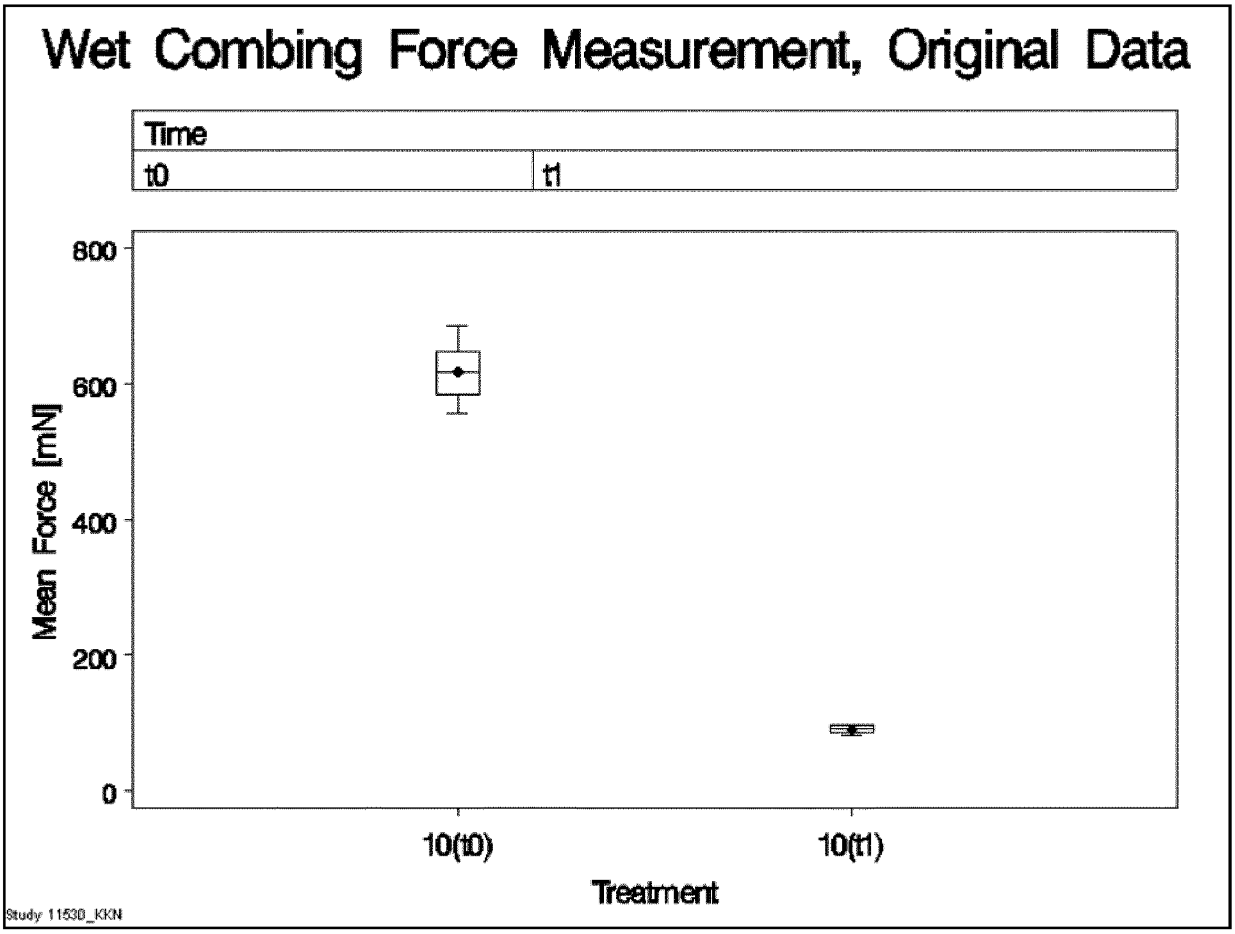


Der Punkt in den jeweiligen Boxen entspricht dem Mittelwert (arithmetisch), die Linie in der Box entspricht dem medianen Mittelwert.

Abbildung 1b:

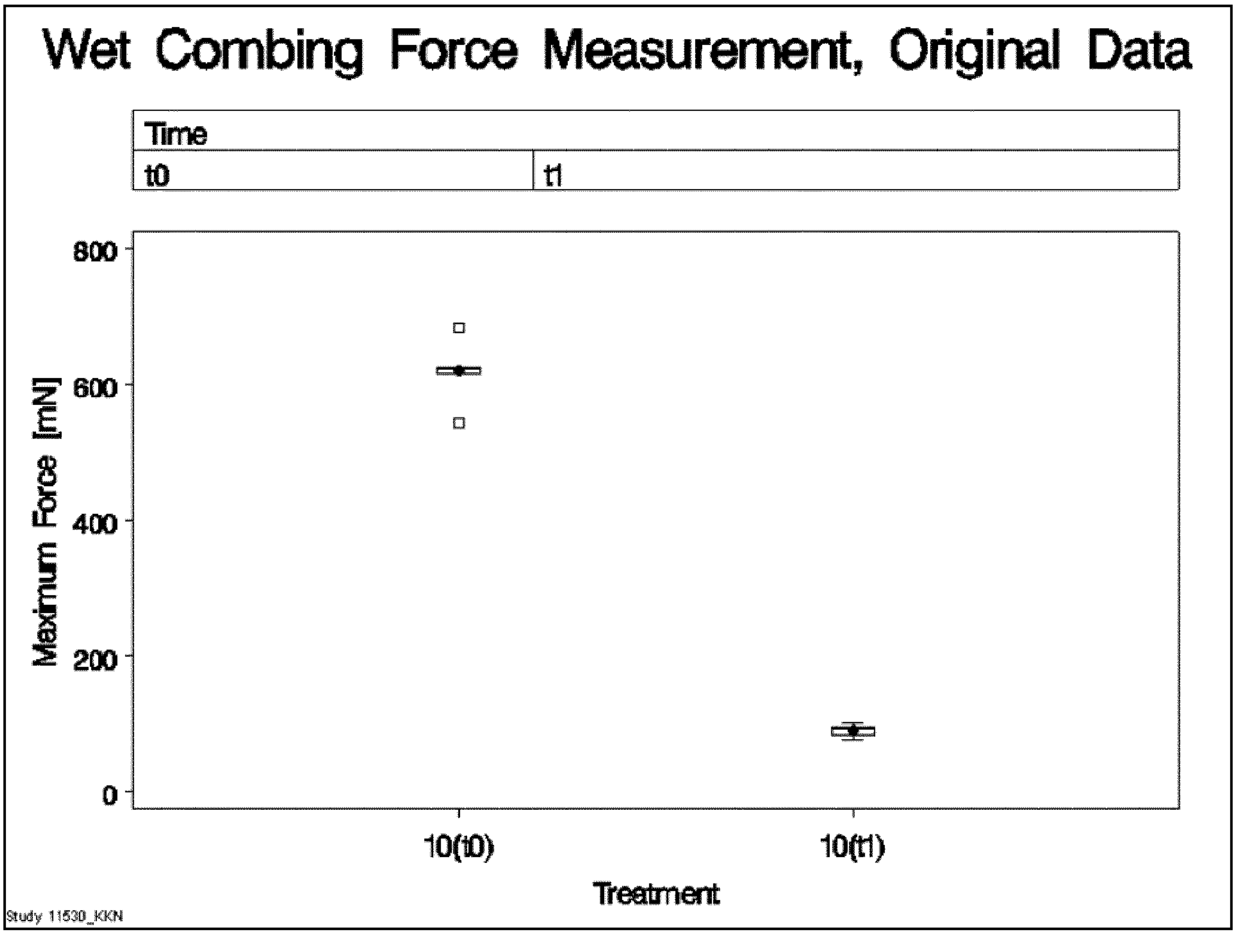


Der Punkt in den jeweiligen Boxen entspricht dem Mittelwert (arithmetisch), die Linie in der Box entspricht dem medianen Mittelwert.

Europäisches Patentamt
European Patent Office
Office européen des brevets

# EUROPÄISCHER RECHERCHENBERICHT

Nummer der Anmeldung
EP 22 18 2769

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2013 226944 A1 (HENKEL AG & CO KGAA) 25. Juni 2015 (2015-06-25)<br>* Absätze [0001], [0006], [0007], [0022], [0030], [0038] *<br>* Absatz [0187]; Beispiel *<br>* Absätze [0042], [0179] *<br>----- | 1-15 | INV.<br>A61Q5/12<br>A61K8/06<br>A61K8/37<br>A61K8/41<br>A61K8/42 |
| | | | RECHERCHIERTE SACHGEBIETE (IPC) |
| | | | A61Q<br>A61K |

Der vorliegende Recherchenbericht wurde für alle Patentansprüche erstellt

1

| Recherchenort | Abschlußdatum der Recherche | Prüfer |
|---|---|---|
| München | 9. Dezember 2022 | Simon, Frédéric |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.....................
& : Mitglied der gleichen Patentfamilie, übereinstimmendes Dokument

EPO FORM 1503 03.82 (P04C03)

# ANHANG ZUM EUROPÄISCHEN RECHERCHENBERICHT ÜBER DIE EUROPÄISCHE PATENTANMELDUNG NR.

EP 22 18 2769

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-12-2022

| Im Recherchenbericht angeführtes Patentdokument | Datum der Veröffentlichung | Mitglied(er) der Patentfamilie | Datum der Veröffentlichung |
|---|---|---|---|
| DE 102013226944 A1 | 25-06-2015 | DE 102013226944 A1 | 25-06-2015 |
| | | EP 3082741 A1 | 26-10-2016 |
| | | US 2016296441 A1 | 13-10-2016 |
| | | WO 2015091049 A1 | 25-06-2015 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2783677 A2